# EUROPÄISCHE PATENTSCHRIFT

(11) **EP 0 772 708 B1**
(45) Veröffentlichungstag und Bekanntmachung des Hinweises auf die Patenterteilung: **30.05.2001**
(21) Anmeldenummer: 95926864.0
(22) Anmeldetag: 12.07.1995
(51) Int. Cl.: D06L 3/12, D06P 3/24, D06P 3/26

(54) **VERFAHREN ZUM OPTISCHEN AUFHELLEN VON POLYAMIDEN**
METHOD OF OPTICALLY BRIGHTENING POLYAMIDES
PROCEDE D'ECLAIRCISSEMENT OPTIQUE DES POLYAMIDES

(30) Priorität: 22.07.1994 DE 4426004
(43) Veröffentlichungstag der Anmeldung: 14.05.1997
(73) Patentinhaber: BASF AKTIENGESELLSCHAFT, 67056 Ludwigshafen (DE)
(72) Erfinder: LEPPERT, Norbert, D-67346 Speyer (DE); WEBER, Dieter, D-67454 Hassloch (DE); HERRMANN, Manfred, D-67061 Ludwigshafen (DE); SCHWINDT, Hans, D-67551 Worms (DE)
(86) Internationale Anmeldenummer: EP9502730
(87) Internationale Veröffentlichungsnummer: WO9603543

(56) Entgegenhaltungen:
- EP-A- 0 017 618
- CH-A- 540 388
- DE-A- 2 427 404
- DE-A- 3 125 495
- DE-A- 4 330 968
- FR-A- 1 415 977
- DATABASE WPI Section Ch, Week 7510 Derwent Publications Ltd., London, GB; Class A23, AN 75-16945W & JP,B,50 003 813 ( TORAY INDS INC) , 10.Februar 1975

## Beschreibung

Die vorliegende Erfindung betrifft ein neues Verfahren zum optischen Aufhellen von synthetischen Polyamiden in textiler Form mit optischen Aufhellern, aus der Klasse der Bisstyrylbenzole.

Das optische Aufhellen von Polyamiden ist bekannt. In der Regel wendet man dabei wasserlösliche optische Aufheller aus der Klasse der Aminocumarine, Diaminostilbene, Pyrazoline, sauren Bisstyrylbiphenyle oder Bistriazolylstilbene an.

Die EP-A 17 618 beschreibt ein Verfahren zum optischen Aufhellen von Polyesterfasern im Ausziehverfahren. Aus der DE-A-3 125 495 ist die Verwendung von Farbstoffen zur Nuancierung von optischen Aufhellern bekannt.

Aufgabe der vorliegenden Erfindung war es, ein neues Verfahren zum optischen Aufhellen von Polyamiden bereitzustellen, das von optischen Aufhellern anderer Klassen ausgeht und mittels dessen man zu aufgehellten Polyamiden mit vorteilhaften anwendungstechnischen Eigenschaften gelangt.

Es wurde nun gefunden, daß das optische Aufhellen von synthetischen Polyamiden in textiler Form durch Behandlung der Polyamide mit optischen Aufhellern in wäßriger Flotte vorteilhaft gelingt, wenn man als optische Aufheller eine oder mehrere Cyanostyrylverbindungen der Formel I 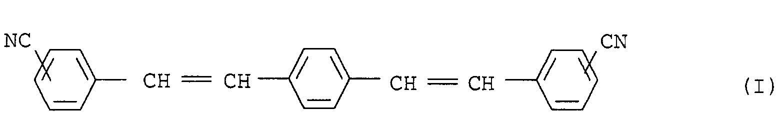 verwendet und die Behandlung in Gegenwart eines oder mehrerer blauer oder violetter Nuancierfarbstoffe vornimmt, wobei man jeweils bezogen auf das Gewicht des aufzuhellenden Polyamids, 0,005 bis 0,3 Gew.-% optischen Aufheller sowie 0,00005 bis 0,02 Gew.-% blauen oder violetten Nuancierfarbstoff anwendet.

Synthetische Polyamide stammen beispielsweise aus der Nylonreihe, z.B. Nylon 6, Nylon 6,6, Nylon 11 oder Nylon 12.

Die Polyamide liegen dabei in textiler Form vor. Darunter sind in der Regel Fasern, Garne, Zwirne, Maschenware, Webware oder Non-wovens zu verstehen.

Bei den obengenannten optischen Aufhellern handelt es sich um an sich bekannte Produkte. Sie sind beispielsweise in Ullmann's, Encyclopedia of Industrial Chemistry, 5. Auflage, Band A18, Seiten 156 und 157, beschrieben.

Cyanostyrylverbindungen der Formel II können entweder als Einzelverbindungen oder als Mischung untereinander zur Anwendung gelangen.

Einzelverbindungen gehorchen der Formel 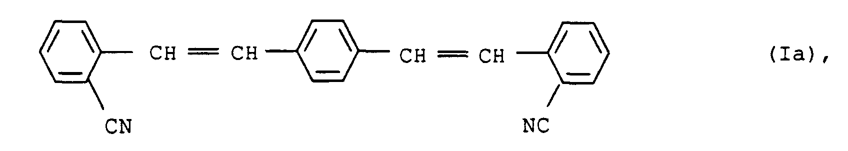 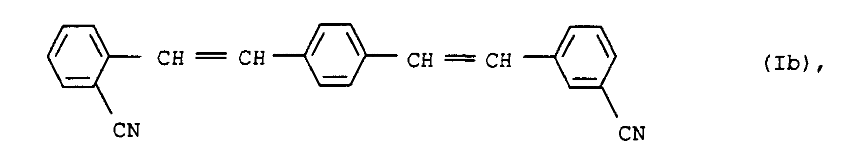 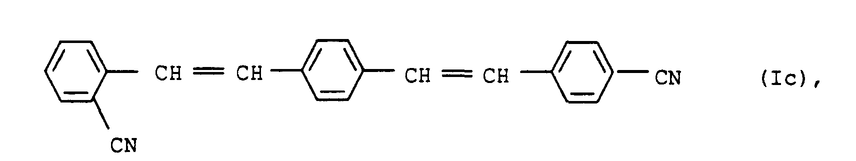 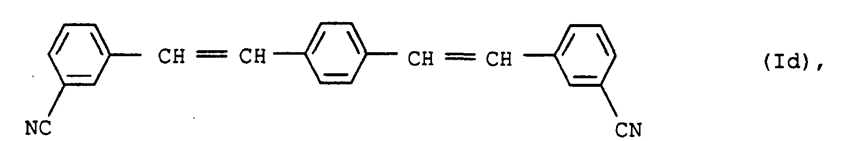 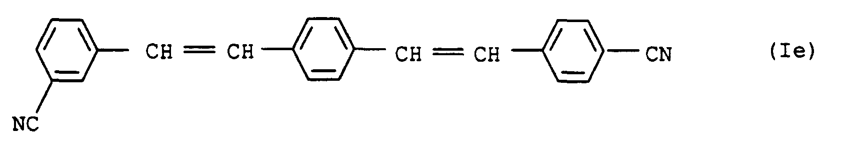 oder

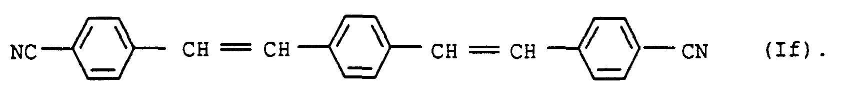

Vorteilhafte Mischungen enthalten z.B. die Einzelverbindungen der Formel Ia, Ib und Ic (Mischung 1), Ib und Ic (Mischung 2), Ia, Ic und Ie (Mischung 3), Ia und Ie (Mischung 4), Ib und Ie (Mischung 5) oder Ic und Ie (Mischung 6).

Hervorzuheben ist auch die Einzelverbindung der Formel Ib, die hervorragende Weißeffekte ergibt. Auch die Einzelverbindung Ic ist hierbei zu nennen.

Weiterhin hervorzuheben sind die Mischungen 1, 2, 3, 4, 5 oder 6, in denen die Einzelverbindungen jeweils im folgenden Gewichtsverhältnis vorliegen.

### Mischung 1:

Ia:Ib:Ic 5:90:5 bis 20:20:60, vorzugsweise ca. 10:60:20.

### Mischung 2:

Ib:Ic 50:50 bis 95:5, vorzugsweise ca. 80:20 oder ca. 90:10.

### Mischung 3:

Ia:Ic:Ie 5:30:65 bis 20:60:20, vorzugsweise ca. 10:40:50.

### Mischung 4:

Ia:Ie 50:50 bis 5:95, vorzugsweise ca. 10:90

### Mischung 5

Ib:Ie 95:5 bis 50:50, vorzugsweise ca. 90:10

### Mischung 6

Ic:Ie 95:5 bis 5:95, vorzugsweise ca. 50:50.

Erfindungsgemäß wird das neue Verfahren in Gegenwart eines oder mehrerer blauer oder violetter Nuancierfarbstoffe vorgenommen. Geeignete Nuancierfarbstoffe stammen in der Regel aus der Klasse der Dispersions- oder Küpenfarbstoffe. Dies sind gebräuchliche Bezeichnungen. Im Colour Index sind solche Farbstoffe z.B. unter der Bezeichnung Disperse Blue oder Disperse Violet oder Vat Blue oder Vat Violet aufgeführt.

Besonders geeignet sind blaue Farbstoffe aus der Klasse der Anthrachinone, Azofarbstoffe, Methinfarbstoffe, Violanthrone oder Indanthrone.

Farbstoffe aus der Klasse der Anthrachinone gehorchen z.B. der Formel 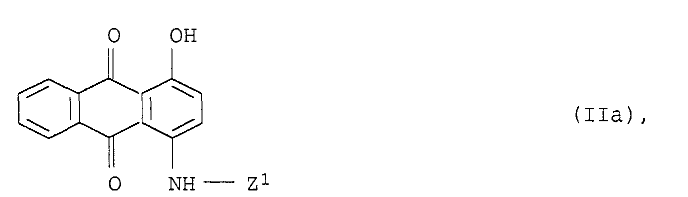 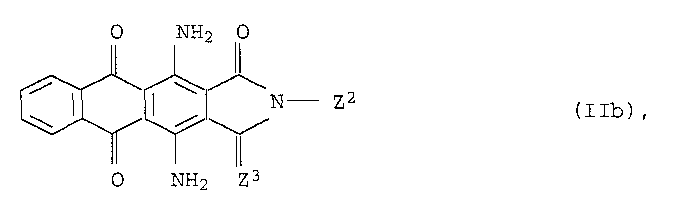 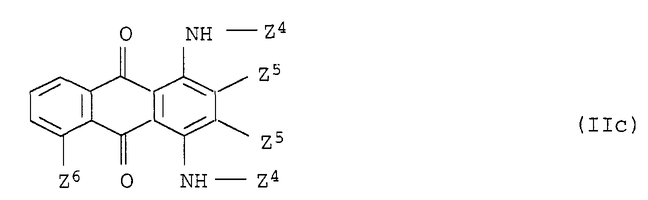 oder 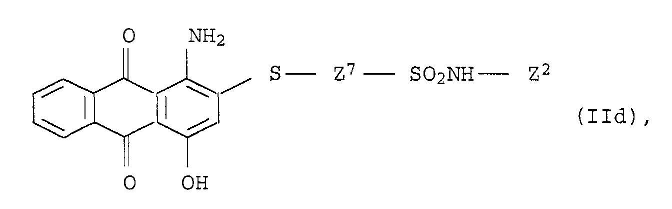 worin
- Z¹: C₁-C₁₀-Alkyl, das gegebenenfalls 1 oder 2 Sauerstoffatome in Etherfunktion unterbrochen ist und durch C₁-C₄-Alkoxycarbonyl oder Cyano substituiert sein kann, oder gegebenenfalls durch C₁-C₄-Alkoxy substituiertes Phenyl,
- Z²: C₁-C₁₀-Alkyl, das gegebenenfalls durch 1 oder 2 Sauerstoffatome in Etherfunktion unterbrochen ist und durch Hydroxy, Phenyl oder C₁-C₈-Alkoxycarbonyl substituiert sein kann
- Z³: Sauerstoff oder Imino,
- Z⁴: Wasserstoff, C₁-C₁₀-Alkyl, das gegebenenfalls durch 1 oder 2 Sauerstoffatome in Etherfunktion unterbrochen ist und durch C₁-C₄-Alkoxycarbonyl oder Cyano substituiert sein kann, oder gegebenenfalls durch C₁-C₄-Alkoxy substituiertes Phenyl,
- Z⁵: Wasserstoff oder Halogen,
- Z⁶: Wasserstoff oder Nitro und
- Z⁷: C₂-C₆-Alkylen oder Phenylen bedeuten.

Bei den obengenannten Farbstoffen handelt es sich im allgemeinen um bekannte Farbstoffe. Farbstoffe der Formel IIb sind z.B. in der US-A-2 628 963, US-A-3 835 154, DE-B-1 266 425 oder
DE-A-2 016 794 beschrieben. Farbstoffe, die der Formel IIa, IIc und IId gehorchen, sind aus z.B. K. Venkataraman, "The Chemistry of Synthetic Dyes", Band 3, Seiten 391 bis 423, 1970, bekannt.

Geeignete Azofarbstoffe sind insbesondere Monoazofarbstoffe mit einer Diazokomponente aus der Anilin- oder heterocyclischen Reihe und einer Kupplungskomponente aus der Anilin- oder heterocyclischen Reihe.

Geeignete Heterocyclen von denen sich die Diazokomponenten ableiten, stammen z.B. aus der Klasse der Aminothiophene, Aminothiazole, Aminoisothiazole, Aminothiadiazole oder Aminobenzisothiazole.

Geeignete Heterocyclen von denen sich die Kupplungskomponenten ableiten, stammen z.B. aus der Klasse der Thiazole oder Diaminopyridine.

Insbesondere entsprechen solche Azofarbstoffe der Formel 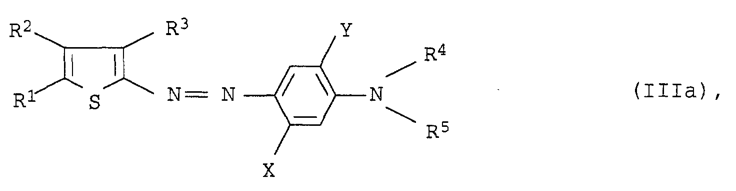 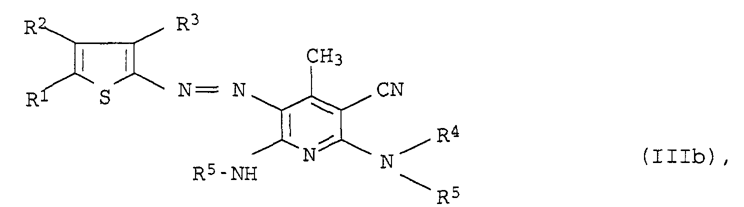 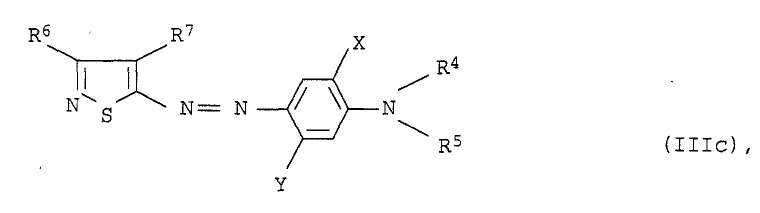 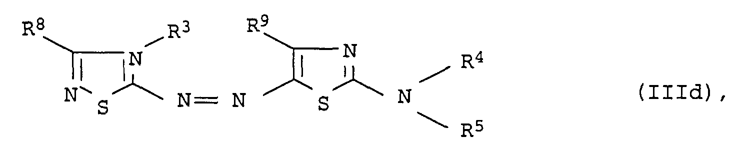 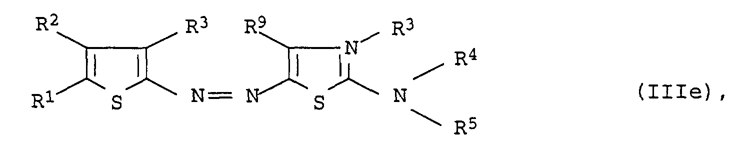 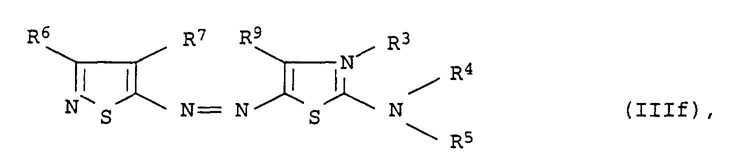 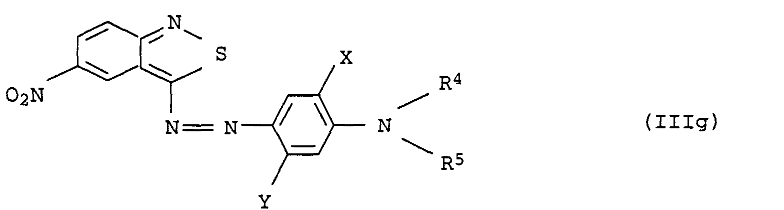 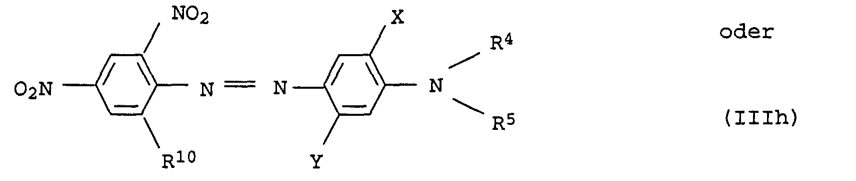 worin
- R¹: Formyl, Cyano, C₁-C₄-Alkoxycarbonyl oder Phenylsulfonyl,
- R²: Wasserstoff, Halogen, C₁-C₈-Alkoxy, Phenoxy, C₁-C₆-Alkylthio, Phenylthio, C₁-C₄-Alkylsulfonyl, Phenylsulfonyl, Methyl oder gegebenenfalls durch Chlor, Methoxy, Ethoxy oder Methyl substituiertes Phenyl,
- R³: Cyano, C₁-C₆-Alkoxycarbonyl, dessen Alkylkette gegebenenfalls durch 1 oder 2 Sauerstoffatome in Etherfunktion unterbrochen ist, Carbamoyl oder C₁-C₄-Mono- oder Dialkylcarbamoyl,
- R⁴ und R⁵: unabhängig voneinander jeweils C₁-C₈-Alkyl, das gegebenenfalls durch 1 bis 3 Sauerstoffatome in Etherfunktion unterbrochen ist und durch Hydroxy, Cyano, Chlor, Phenyl, C₁-C₆-Alkanyloxy, C₁-C₆-Alkoxycarbonyl, C₁-C₆-Alkoxycarbonyloxy oder C₁-C₆-Mono- oder Dialkylaminocarbonyloxy substituiert sein kann, oder C₃-C₄-Alkenyl oder R⁴ auch Wasserstoff,
- X: Wasserstoff, C₁-C₄-Alkyl, C₁-C₄-Alkoxy, Chlor, Brom oder einen Rest der Formel -NH-CO-R¹¹, wobei R¹¹ für C₁-C₄-Alkyl, das durch C₁-C₄-Alkoxy, Cyano, Hydroxy, Chlor oder C₁-C₄-Alkanoyloxy substituiert sein kann, oder C₂-C₃-Alkenyl steht,
- Y: Wasserstoff, C₁-C₄-Alkyl oder C₁-C₄-Alkoxy
- R⁶: gegebenenfalls durch C₁-C₄-Alkoxy substituiertes C₁-C₄-Alkyl, gegebenenfalls durch C₁-C₄-Alkoxy substituiertes Benzyl, gegebenenfalls durch Chlor, Methyl, Methoxy oder Ethoxy substituiertes Phenyl, Pyrid-2-yl oder Pyrid-3-yl,
- R⁷: Cyano, Chlor oder Brom,
- R⁸: gegebenenfalls durch C₁-C₄-Alkoxy, Chlor oder Cyano substituiertes C₁-C₆-Alkyl, gegebenenfalls durch C₁-C₄-Alkoxy, Chlor oder Cyano substituiertes Phenyl, gegebenenfalls durch Cyano oder C₁-C₄-Alkoxycarbonyl substituiertes C₁-C₆-Alkylthio oder gegebenenfalls durch Cyano oder C₁-C₄-Alkoxycarbonyl substituiertes Phenylthio,
- R⁹: gegebenenfalls durch C₁-C₄-Alkoxy oder Acetylamino substituiertes Thienyl oder Pyridyl und
- R¹⁰: Cyano, Chlor oder Brom bedeuten.

Die obengenannten Azofarbstoffe sind an sich bekannt. Farbstoffe der Formel IIIa und IIIb sind z.B. in der US-A-5 283 326 oder US-A-5 145 952 beschrieben. Aus der EP-A-87 616, EP-A-87 677, EP-A-121 875, EP-A-151 287 sowie der US-A-4 960 873 sind Farbstoffe der Formel IIIc bekannt. Die US-A-5 132 412 beschreibt Farbstoffe vom Typ der Formel IIId und IIIf. Aus der US-A-5 216 139 sind Farbstoffe der Formel IIIe bekannt. Farbstoffe der Formel IIIg und IIIh sind z.B. in der US-A-3 981 883, DE-A-3 112 427, EP-A-64 221 oder in Venkataraman "The Chemistry of Synthetic Dyes", Band 3, Seiten 444 bis 447, beschrieben oder können nach den dort genannten Methoden erhalten werden.

Geeignete Methinfarbstoffe gehorchen z.B. der Formel IV 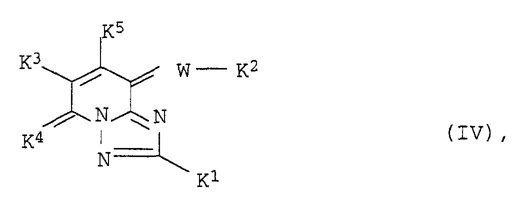 worin
- W: Stickstoff oder CH,
- K¹: C₁-C₂₀-Alkyl, das gegebenenfalls substituiert ist und durch ein oder mehrere Sauerstoffatome in Etherfunktion unterbrochen sein kann, gegebenenfalls substituiertes Phenyl oder Hydroxy,
- K²: einen 5-gliedrigen aromatischen heterocyclischen Rest,
- K³: Wasserstoff, Cyano, Carbamoyl, Carboxyl oder C₁-C₄-Alkoxycarbonyl,
- K⁴: Sauerstoff oder einen Rest der Formel C(CN)₂, C(CN)COOK⁶ oder C(COOK⁶)₂, wobei K⁶ jeweils für C₁-C₈-Alkyl, das gegebenenfalls durch 1 oder 2 Sauerstoffatome in Etherfunktion unterbrochen ist, steht, und
- K⁵: Wasserstoff oder C₁-C₄-Alkyl bedeuten.

Wenn in Formel IV substituierte Alkylreste auftreten, so können als Substituenten, sofern nicht anders vermerkt, z.B. Phenyl, C₁-C₄-Alkylphenyl, C₁-C₄-Alkoxyphenyl, Halogenphenyl, C₁-C₈-Alkanoyloxy, C₁-C₈-Alkylaminocarbonyloxy, C₁-C₂₀-Alkoxycarbonyl, C₁-C₂₀-Alkoxycarbonyloxy, wobei die Alkylkette der drei letztgenannten Reste gegebenenfalls durch 1 bis 4 Sauerstoffatome in Etherfunktion unterbrochen und/oder durch Phenyl oder Phenoxy substituiert ist, Halogen, Hydroxy oder Cyano in Betracht kommen. Die Alkylreste weisen dabei in der Regel 1 oder 2 Substituenten auf.

Wenn in Formel IV Alkylreste auftreten, die durch Sauerstoffatome in Etherfunktion unterbrochen sind, so sind, sofern nicht anders vermerkt, solche Alkylreste bevorzugt, die durch 1 bis 4 Sauerstoffatome, insbesondere 1 bis 2 Sauerstoffatome, in Etherfunktion unterbrochen sind.

Wenn in Formel IV substituierte Phenylreste auftreten, so können als Substituenten, sofern nicht anders vermerkt, z.B. C₁-C₈-Alkyl, C₁-C₈-Alkoxy, Halogen, dabei insbesondere Chlor oder Brom, oder Carboxyl in Betracht kommen. Die Phenylreste weisen dabei in der Regel 1 bis 3 Substituenten auf.

Reste K² können sich z.B. von Komponenten aus Pyrrol-, Thiazol-, Thiophen- oder Indolreihe ableiten.

Wichtige Reste K² sind z.B. solche der Formel 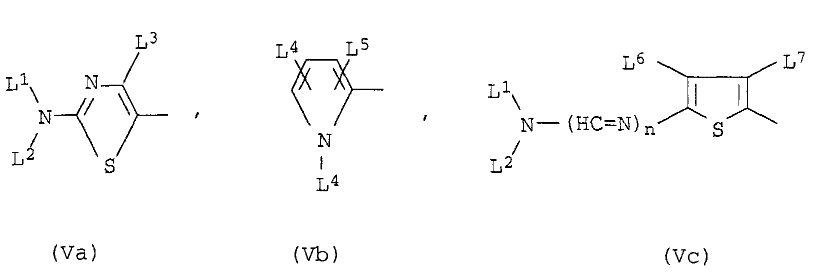 oder 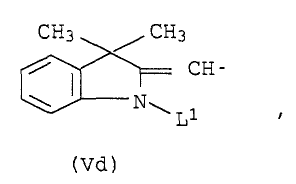 worin
- n: 0 oder 1,
- L¹ und L²: unabhängig voneinander jeweils Wasserstoff oder mit Ausnahme von Hydroxy auch den obengenannten Rest K¹ oder zusammen mit dem sie verbindenden Stickstoffatom einen 5- oder 6-gliedrigen gesättigten heterocyclischen Rest, der gegebenenfalls weitere Heteroatome aufweist,
- L³: Wasserstoff, Halogen, C₁-C₈-Alkyl, gegebenenfalls durch C₁-C₄-Alkyl oder C₁-C₄-Alkoxy substituiertes Phenyl, gegebenenfalls durch C₁-C₄-Alkyl oder C₁-C₄-Alkoxy substituiertes Benzyl, Cyclohexyl, Thienyl, Hydroxy oder C₁-C₈-Monoalkylamino,
- L⁴ und L⁵: unabhängig voneinander jeweils Wasserstoff, Hydroxy, gegebenenfalls durch Phenyl oder C₁-C₄-Alkylphenyl substituiertes C₁-C₈-Alkyl, gegebenenfalls durch Phenyl oder C₁-C₄-Alkylphenyl substituiertes C₁-C₈-Alkoxy, C₁-C₈-Alkanoylamino, C₁-C₈-Alkylsulfonylamino oder C₁-C₈-Mono- oder Dialkylaminosulfonylamino,
- L⁶: Cyano, Carbamoyl, C₁-C₈-Mono- oder Dialkylcarbamoyl, C₁-C₈-Alkoxycarbonyl oder gegebenenfalls substituiertes Phenyl und
- L⁷: Halogen, Wasserstoff, C₁-C₄-Alkyl, C₁-C₄-Alkoxy, C₁-C₄-Alkylthio, gegebenenfalls durch C₁-C₄-Alkyl oder C₁-C₄-Alkoxy substituiertes Phenyl oder Thienyl bedeuten.

Solche Methinfarbstoffe sind z.B. in der älteren Patentanmeldung PCT/EP 95/00327 beschrieben.

Geeignete Violanthronfarbstoffe gehorchen z.B. der Formel VI 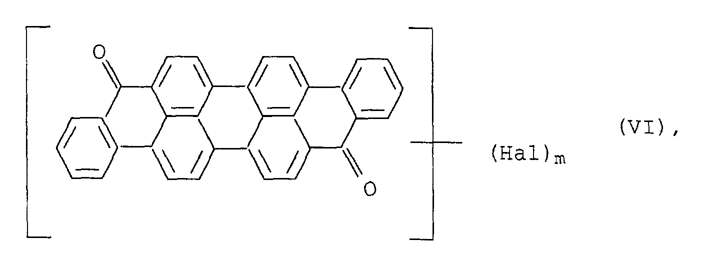 in der Hal Chlor oder Brom und m 1 oder 2 bedeuten.

Solche Farbstoffe sind z.B. als C.I. Vat Violet 9 (60005) oder C.I. Vat Violet 1 (60010) bekannt.

Ein geeigneter Indanthronfarbstoff gehorcht z.B. der Formel VII

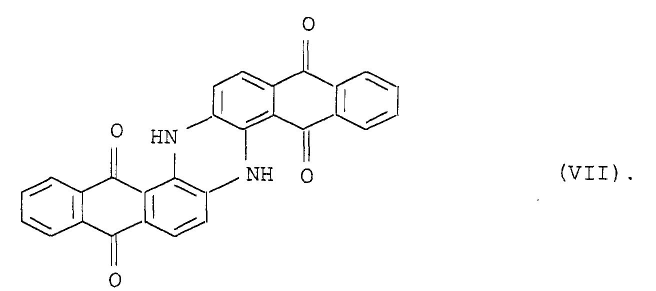

Er ist unter der Bezeichnung Indanthron oder C.I. Vat Blue 4 (69800) bekannt.

Von besonderer Bedeutung ist eine Verfahrensweise, in der man die Behandlung der Polyamide in Gegenwart eines oder mehrerer blauer oder violetter Nuancierfarbstoffe aus der Klasse der Anthrachinone, insbesondere solchen der Formel III, und/oder der Violanthrone, insbesondere solchen der Formel VI, vornimmt, wobei der Farbstoff der Formel VI, in der Hal Brom und n 1 bedeutet, besonders hervorzuheben ist.

Technisch besonders wichtig ist die Durchführung des Verfahrens in Gegenwart des Farbstoffs der Formel IIIe 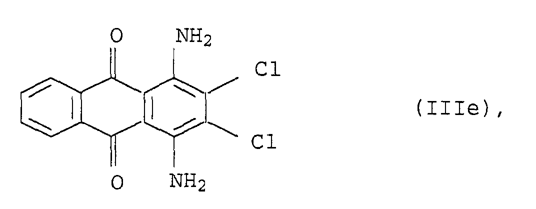 der auch unter dem Namen C.I. Disperse Violet 28 (61102) bekannt ist, oder einer Mischung aus einem Farbstoff der Formel IIIf, 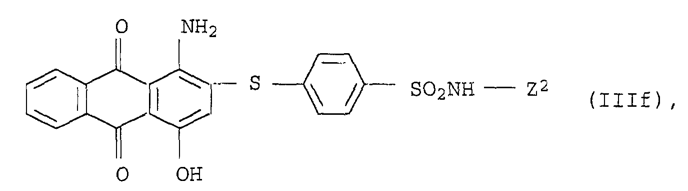 worin Z² die obengenannte Bedeutung besitzt und insbesondere 4-Oxahexyl bedeutet, und einem Farbstoff der Formel VI, insbesondere mit Hal = Brom und n = 1.

Bezogen auf das Gewicht des aufzuhellenden Polyamids wendet man vorzugsweise 0,01 bis 0,1 Gew.%, optischen Aufheller sowie 0,0005 bis 0,002 Gew.%, blauen oder violetten Nuancierfarbstoff an.

Die Aufhellung der Polyamide erfolgt in der Regel nach dem Auszieh- oder Thermosolverfahren.

Beim Ausziehverfahren wird in der Regel das aufzuhellende Polyamid bei einer Temperatur von 10 bis 35°C in ein Färbebad gebracht, das den optischen Aufheller, den blauen oder violetten Nuancierfarbstoff und gegebenenfalls Hilfsmittel, z.B. Dispergiermittel, Carbonsäuren oder Alkalispender, enthält und dessen pH-Wert 5 bis 12, vorzugsweise 7 bis 11, beträgt. Das Flottenverhältnis (Gewichtsverhältnis Polyamid:Flotte) beträgt dabei 1:3 bis 1:40, vorzugsweise 1:5 bis 1:20. Das Bad wird dann innerhalb von 15 bis 30 Minuten auf eine Temperatur von 90 bis 130°C, vorzugsweise 95 bis 100°C, erhitzt und 15 bis 60 Minuten bei dieser Temperatur gehalten. Danach wird das aufgehellte Polyamid gespült und getrocknet.

Beim Thermosolverfahren wird üblicherweise das aufzuhellende Polyamid mit einer wäßrigen Flotte, die den optischen Aufheller, den blauen oder violetten Nuancierfarbstoff und gegebenenfalls Hilfsmittel (s.o.) enthält, foulardiert. Die Flottenaufnahme beträgt im allgemeinen 40 bis 70%. Danach wird das Polyamid getrocknet und bei einer Temperatur von 150 bis 200°C für 5 bis 60 Sekunden fixiert.

Im erfindungsgemäßen Verfahren werden optische Aufheller und Nuancierfarbstoffe in der Regel als wäßrige Zubereitung zur Anwendung gebracht. Solche Zubereitungen enthalten in der Regel Wasser und, jeweils bezogen auf das Gewicht der Zubereitung, 1 bis 40 Gew.%, vorzugsweise 3 bis 10 Gew.%, der oben näher bezeichneten Mischung aus Aufheller und Nuancierfarbstoff sowie 5 bis 60 Gew.%, vorzugsweise 5 bis 52 Gew.%, an Hilfsmitteln.

Geeignete Hilfsmittel sind z.B. anionische oder nichtionische Dispergiermittel, aus der Klasse der Ethylenoxidaddukte mit Fettalkoholen, höheren Fettsäuren oder Alkylphenolen oder Ethylendiamin-Ethylenoxid-Propylenoxidaddukte, oder Dispergiermittel, wie in der DE-A-2 745 449 beschrieben, Copolymerisate von N-Vinylpyrrolidon mit 3-Vinylpropionsäure, Wasserrückhaltemittel, wie Ethylenglykol, Glycerin oder Sorbit, oder Biocide.

In einer bevorzugten Verfahrensweise verwendet man eine Aufhellerzubereitung enthaltend neben Wasser, jeweils bezogen auf das Gewicht der Zubereitung, 1 bis 40 Gew.%, vorzugsweise 3 bis 10 Gew.%, der oben näher bezeichneten Mischung aus Aufheller und Nuancierfarbstoff, 3 bis 12 Gew.% anionisches oder nichtionisches Dispergiermittel, 1 bis 15 Gew.% Copolymerisate von N-Vinylpyrrolidon mit 3-Vinylpropionsäure und 1 bis 25 Gew.% weiterer Hilfsmittel (z.B. Wasserrückhaltemittel oder Biocide).

Die aufzuhellenden Polyamide können auch in Mischung mit anderen gebräuchlichen Materialien, z.B. Cellulose, Polyurethane oder Polyester, vorliegen.

Mittels des erfindungsgemäßen Verfahrens gelingt es, Polyamide sowohl im Auszieh- als auch im Thermosolverfahren mit hervorragenden Weißeffekten aufzuhellen. Die aufgehellten Materialien zeigen gute Gebrauchsechtheiten.

Die folgenden Beispiele sollen die Erfindung näher erläutern.

### Färbevorschrift

a) Ausziehverfahren
   Polyamidgewebe wird bei 25°C in ein Färbebad eingebracht, das X Gew.-% Aufheller/Nuancierfarbstoff-Mischung (bezogen auf das Gewicht des Gewebes) und 1 g/l des Natriumsalzes eines Kondensationsproduktes aus Naphthalin-2-sulfonsäure und Formaldehyd enthält und dessen pH-Wert auf den unten näher bezeichneten pH-Wert gestellt wurde. Das Bad wird dann innerhalb von 30 Minuten auf 100°C erhitzt und noch 30 Minuten bei dieser Temperatur gehalten. Danach wird das Gewebe gespült und getrocknet.
b) Thermosolverfahren
   Man foulardiert bei Raumtemperatur Polyamidgewebe mit einer wäßrigen Flotte, enthaltend X g/l Aufheller/Nuancierfarbstoff-Mischung und Y g Soda. Die Flottenaufnahme beträgt 60 %. Anschließend wird das Gewebe getrocknet und danach bei 190°C für 30 Sekunden fixiert.

### Beispiel 1

Mischung aus 50 g Aufheller der Formel 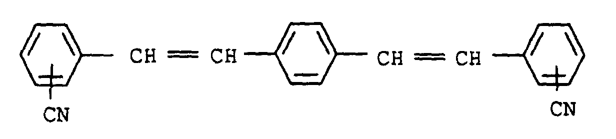 (Aufhellermischung - Stellung der Cyanogruppe zur Vinylgruppe: ortho-meta 60 Gew.%, ortho-para 26 Gew.% und ortho-ortho 14 Gew.%),
0,5 g Farbstoff der Formel 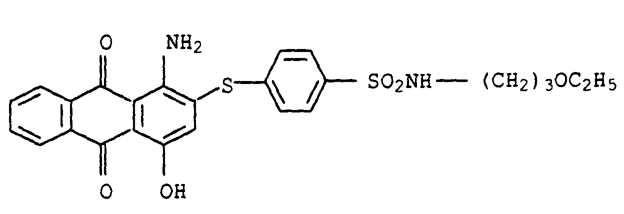 und 0,5 g Farbstoff der Formel

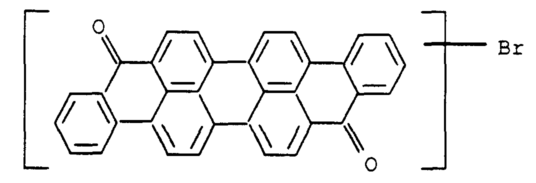

Thermosolverfahren
X = 1 g/l
Y = 1 g/l

### Beispiel 2

Man verwendete die in Beispiel 1 beschriebene Aufheller/Farbstoff-Mischung.

Ausziehverfahren
X = 0,1 Gew.%
pH-Wert: 10

### Beispiel 3

Mischung aus 100 g Aufheller der Formel 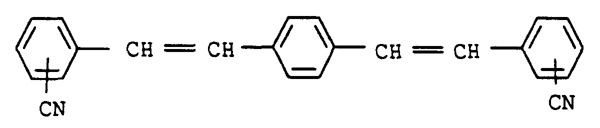 (Aufhellermischung - Stellung der Cyanogruppe zur Vinylgruppe: ortho-meta 80 Gew.% und ortho-para 20 Gew.%),
0,5 g Farbstoff der Formel 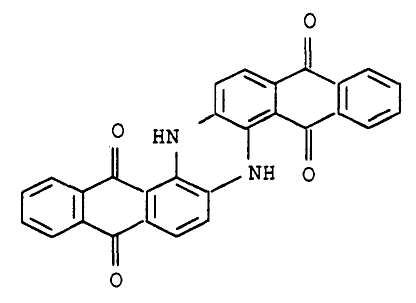 und 0,7 g Farbstoff der Formel

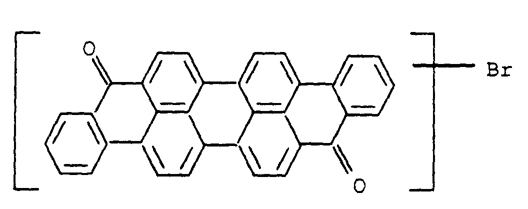

Thermosolverfahren
X = 0,5 g/l
Y = 0 g/l

### Beispiel 4

Man verwendete die in Beispiel 3 beschriebene Aufheller/Farbstoff-Mischung.

Ausziehverfahren
X = 0,05 Gew.%
pH-Wert: 10

### Beispiel 5

Mischung aus 100 g Aufheller der Formel 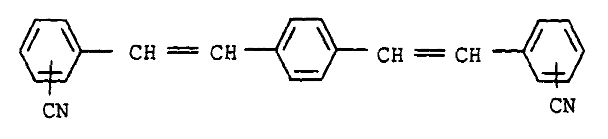 (Aufhellermischung - Stellung der Cyanogruppe zur Vinylgruppe: ortho-para 40 Gew.%, meta-para 50 Gew.% und ortho-ortho 10 Gew.%) und
1 g Farbstoff der Formel

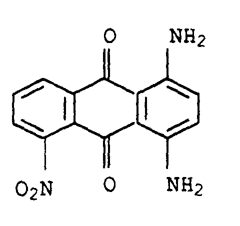

Thermosolverfahren
X = 1 g/l
Y = 0 g/l

### Beispiel 6

Man verwendete die in Beispiel 5 beschriebene Aufheller/Farbstoff-Mischung.

Ausziehverfahren
X = 0,1 Gew.%
pH-Wert: 7

## Patentansprüche

1. Verfahren zum optischen Aufhellen von synthetischen Polyamiden in textiler Form durch Behandlung der Polyamide mit optischen Aufhellern in wäßriger Flotte, dadurch gekennzeichnet, daß man als optische Aufheller eine oder mehrere Cyanostyrylverbindungen der Formel I 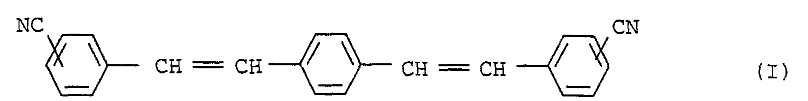 verwendet und die Behandlung in Gegenwart eines oder mehrerer blauer oder violetter Nuancierfarbstoffe vornimmt, wobei man jeweils bezogen auf das Gewicht des aufzuhellenden Polyamids, 0,005 bis 0,3 Gew.% optischen Aufheller sowie 0,00005 bis 0,02 Gew.% blauen oder violetten Nuancierfarbstoff anwendet.

2. Verfahren nach Anspruch 1, dadurch gekennzeichnet, daß die blauen oder violetten Nuancierfarbstoffe aus der Klasse der Anthrachinone, Azofarbstoffe, Methinfarbstoffe, Violanthrone oder Indanthrone stammen.

## Claims

1. A process for the optical brightening of synthetic polyamides in textile form by treatment of the polyamides with optical brighteners in an aqueous liquor, characterized in that the optical brighteners used comprise one or more cyanostyryl compounds of the formula I 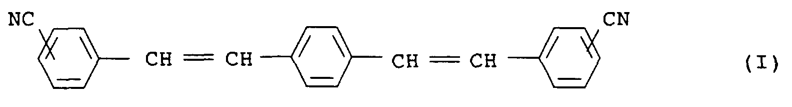 and the treatment is effected in the presence of one or more blue or violet shading dyes, using, in each case based on the weight of the polyamide to be brightened, from 0.005 to 0.3% by weight of optical brightener and from 0.00005 to 0.02% by weight of blue or violet shading dye.

2. A process as claimed in claim 1 characterized in that the blue or violet shading dyes belong to the class of the anthraquinones, azo dyes, methine dyes, violanthrones or indanthrones.

## Revendications

1. Procédé pour azurer optiquement des polyamides synthétiques sous forme textile en traitant les polyamides avec des azurants optiques dans un bain aqueux, caractérisé en ce que l'on utilise un ou plusieurs composés cyanostyryle de formule I 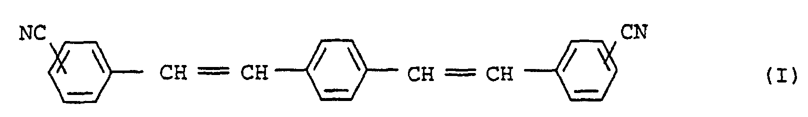 en tant qu'azurants optiques et on procède au traitement en présence d'un ou plusieurs colorants de nuançage bleus ou violets, dans lequel on utilise respectivement 0,005 à 0,3% en poids d'azurant optique ainsi que 0,00005 à 0,02% en poids de colorants de nuançage bleus ou violets, par rapport au poids du polyamide à azurer.

2. Procédé selon la revendication 1, caractérisé en ce que les colorants de nuançage bleus ou violets proviennent de la classe des anthraquinones, des colorants azoïques, des colorants méthine, des violanthrones ou indanthrones.
